# EUROPÄISCHE PATENTANMELDUNG

(11) **EP 0 953 566 A2**
(43) Veröffentlichungstag der Anmeldung: **03.11.1999**
(21) Anmeldenummer: 99106914.7
(22) Anmeldetag: 08.04.1999
(51) Int. Cl.: C07D 213/127, C07D 213/64

(54) **Selektive Umsetzung von Pyridinen mit Elektrophilen**

(30) Priorität: 27.04.1998 DE 19818681
(71) Anmelder: BASF AKTIENGESELLSCHAFT, 67056 Ludwigshafen (DE)
(72) Erfinder: Gypser, Andreas, Dr., 68159 Mannheim (DE); Sauter, Hubert, Dr., 68167 Mannheim (DE); Ptock, Arne, Dr., 67067 Ludwigshafen (DE); Stürmer, Rainer, Dr., 67127 Rödersheim-Gronau (DE); Grammenos, Wassilios, Dr., 67063 Ludwigshafen (DE)

(57) **Zusammenfassung**

Die Erfindung betrifft ein Verfahren zur selektiven Umsetzung eines Pyridins der Formel I, 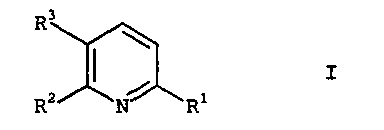 in der die Substituenten die in der Beschreibung genannte Bedeutung haben,
mit einem Elektrophil der Formel III,

E-L¹ III

in der E die Bedeutung C₁-C₆-Alkyl, Brom oder Iod hat und L¹ für eine Abgangsgruppe steht, zu entweder
a) einem in 3-Stellung substituierten Pyridin der Formel IIa, 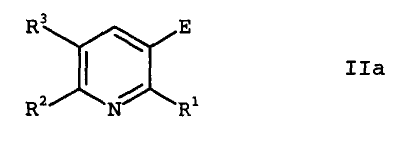 oder
b) einem in 4-Stellung substituierten Pyridin der Formel IIb.

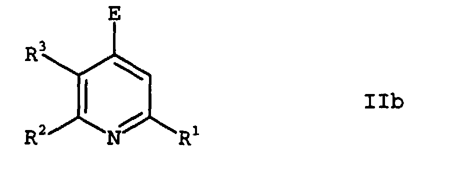

## Beschreibung

Die Erfindung betrifft ein Verfahren zur selektiven Umsetzung eines Pyridins der Formel I, 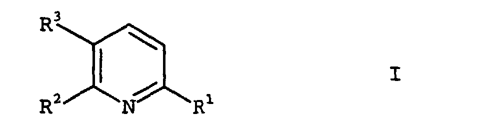 in der die Substituenten die folgende Bedeutung haben:
- R¹: einen Rest OR⁴, Chlor oder C₁-C₄-Alkylcarbonylamino;
- R²: Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
- R³: perhalogeniertes C₁-C₂-Alkyl oder einen Rest R⁵(C=O)-;
- R⁴: C₁-C₆-Alkyl, C₁-C₄-Alkoxy-C₁-C₂-alkyl, Di-(C₁-C₄-alkyl) aminocarbonyl, Phenyl oder Benzyl, wobei der Phenyl- und Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
- R⁵: Wasserstoff, perhalogeniertes C₁-C₂-Alkyl, C₁-C₆-Alkoxy oder Di-(C₁-C₄-alkyl) amino;
mit einem Elektrophil der Formel III,

E-L¹ III

in der E die Bedeutung C₁-C₆-Alkyl, Brom oder Iod hat und L¹ für eine Abgangsgruppe wie Chlorid, Bromid, Iodid oder im Fall, daß E C₁-C₆-Alkyl bedeutet, zusätzlich für C₁-C₆-Alkylsulfonat, Arylsulfonat oder Mono-C₁-C₄-Alkylsulfat steht, zu entweder
a) einem in 3-Stellung substituierten Pyridin der Formel IIa, 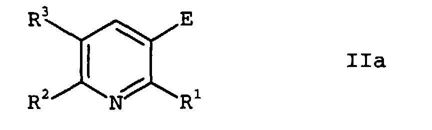 oder
b) einem in 4-Stellung substituierten Pyridin der Formel IIb.

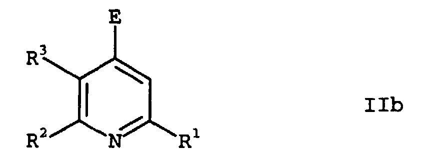

Aus der Literatur sind verschieden Methoden zur Einführung eines Elektrophils in 3- bzw. 4-Position am Pyridinring bekannt.

Zur gezielten Herstellung von 2-Methoxy-3-methyl-5-(trifluormethyl)pyridin wird in EP-A 316 185 von 3-Brom-2-methoxy-5-(trifluormethyl)pyridin ausgegangen. Via Halogen/Metall-Austausch und anschließender Umsetzung mit Methyliodid kann das 3-Methylpyridin gewonnen werden. Ein wesentlicher Nachteil dieses Verfahrens ist, daß von teuren und zum Teil nur schwer zugänglichen Brompyridinen ausgegangen wird.

Die direkte Metallierung in 3- bzw. 4-Position am Pyridinring verläuft nur in Einzelfällen regioselektiv, sodaß bei der anschließenden Umsetzung mit Elektrophilen in der Regel Stoffgemische entstehen. Aus Liebigs Ann., 1995 S.1441 bis 1446 ist ein Verfahren zur selektiven Metallierung und anschließenden Umsetzung mit Elektrophilen bekannt. Die Regiochemie wird in diesem Verfahren in erster Linie von sterischen Effekten gelenkt. Selbst geringe Änderungen der Substituenten in 2- oder 5-Stellung beeinflussen die Regioselektivität des Metallierungsschrittes.

Aufgabe der vorliegenden Erfindung war es demnach, ein Verfahren zu finden, das die genannten Nachteile nicht aufweist und mit Hilfe dessen es gelingt, die Pyridine der Formel I mit den Elektrophilen der Formel III selektiv und in guten Ausbeuten zum gewünschten in 3-Stellung substituierten Pyridin IIa bzw. zu den in 4-Stellung subst. Pyridin IIb umzusetzen.

Demgemäß wurde das eingangs erwähnte Verfahren gefunden, das dadurch gekennzeichnet ist, daß
a) das Pyridin I zunächst mit einer Lithiumbase der Formel IV, 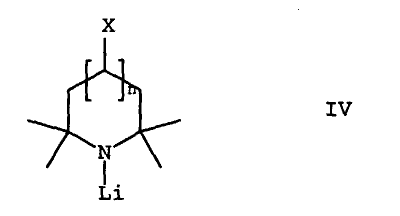 in der n für 0 oder 1 und X für Wasserstoff oder C₁-C₄-Alkoxy stehen, und anschließend mit einem Elektrophil der Formel III zu einem in 3-Stellung substituierten Pyridin der Formel IIa, oder
b) das Pyridin I zunächst mit Lithiumdiisopropylamid und anschließend einem Elektrophil der Formel III zu einem in 4-Stellung substituierten Pyridin der Formel IIb umgesetzt wird.

Die Regiochemie läßt sich alleine durch die Wahl der verwendeten Lithiumbase im Metallierungsschritt steuern. Während beispielsweise mit Lithiumtetramethylpiperidid (LTMP) nahezu ausschließlich in 3-Stellung substituierte Pyridine IIa erhalten werden, werden mit Lithiumdiisopropylamid (LDA) 4-substituierte Pyridine IIb in hoher Selektivität gewonnen.

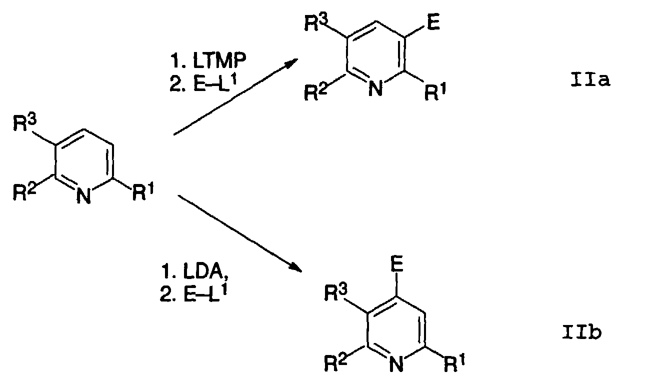

Eine bevorzugte Ausführungsform des Verfahrens ist die selektive Alkylierung der Pyridine I mit einem Alkylierungsmittel III' zu den 3-Alkylpyridinen IIa' und 4-Alkylpyridinen IIb'.

Darüberhinaus wurden nach diesen Verfahren erhältliche, neue 3-Alkylpyridine IIa' und 4-Alkylpyridine IIb' gefunden, die sich als Zwischenprodukte zur Synthese von Pharmawirkstoffen und Wirkstoffen mit pestiziden Eigenschaften wie sie aus der EP-A 398 692 bekannt sind eignen.

Im weiteren wird das erfindungsgemäße Verfahren näher erläutert.

Als Ausgangsstoffe für das Verfahren kommen die Pyridine I in Frage, wobei die Substituenten die folgende Bedeutung haben:
- R¹: einen Rest OR⁴, Chlor oder C₁-C₄-Alkylcarbonylamino;
- R²: Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
- R³: perhalogeniertes C₁-C₂-Alkyl oder einen Rest R⁵(C=O)-;
- R⁴: C₁-C₆-Alkyl, C₁-C₄-Alkoxy-C₁-C₂-alkyl, Di-(C₁-C₄-alkyl)aminocarbonyl, Phenyl oder Benzyl, wobei der Phenyl- und Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
- R⁵: Wasserstoff, perhalogeniertes C₁-C₂-Alkyl, C₁-C₆-Alkoxy oder Di-(C₁-C₄-alkyl) amino.

Bevorzugt werden Pyridine I eingesetzt, wobei die Substituenten R¹ bis R⁵ für sich allein genommen oder in Kombination die folgenden Bedeutungen aufweisen:
- R¹: einen Rest OR^{4;}
- R²: Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
- R³: perfluoriertes C₁-C₂-Alkyl;
- R⁴: C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
- R⁵: C₁-C₆-Alkyl, perhalogeniertes C₁-C₂-Alkyl, C₁-C₄-Alkoxy oder Di-C₁-C₄-alkylamino;

Besonders bevorzugt werden Pyridine I eingesetzt, wobei die Substituenten R¹ bis R⁵ für sich allein genommen oder in Kombination die folgenden Bedeutungen aufweisen:
- R¹: Methoxy oder Benzyloxy;
- R²: Wasserstoff, Fluor, Chlor, einen Rest OR⁴ ;
- R³: CF₃;
- R⁴: C₁-C₆-Alkyl, unsubstitiertes oder im Phenylteil substituiertes Benzyl.

Die Pyridine I lassen sich nach literaturbekannten Verfahren herstellen (Houben-Weyl Bd. E 7b, 286-1004, DE-A 27 555 36 (Pyridine allgemein); Tetradedron Lett. (1995) 4571 (Halogenpyridine); Chem. Rev. 97 (1997) 757-786, J. Chem. Soc. Perkin I (1989) 283-287, Heterocycles 194 (22) 117, US 4,833,250, US 4,634,771, US 4,745,193, US 4,680,406 (Halogenalkylpyridine); JP 86/034418, J. Org. Chem. 55 (1990) S. 69 - 73, Adv. Heterocyclic Chem. 52 (1991) 187 (Alkoxyridine); J. Org. Chem. 48 (1983) 3401 (Alkoxycarbonylaminopyridine).

2-Alkoxysubstituierte Pyridine I lassen sich ausgehend von den entsprechenden 2-Halogenpyridinen und dem jeweiligen Alkohol in Gegenwart von Basen wie beispielsweise Kaliumtert.-butylat oder Natriummethylat unmittelbar herstellen.

Für die Jodierung eignen sich Elektrophile wie z.B. elementares Iod, Iodbromid und Iodchlorid. Für die Bromierung eignen sich-Elektrophile wie beispielsweise elementares Brom oder Bromchlorid.

Als Alkylierungsmittel eignen sich die Verbindungen der Formel III'

A-L¹ III'

in der A für C₁-C₆-Alkyl und L¹ für eine Abgangsgruppe wie Chlorid, Bromid, Iodid, C₁-C₆-Alkylsulfonat, Arylsulfonat oder Mono-C₁-C₆-Alkylsulfat steht. Besonders geeignet sind Alkylierungsmittel, wobei A C₁-C₄-Alkyl und vorzugsweise Methyl oder Ethyl bedeutet. Als Abgangsgruppe wird vorzugweise Chlorid, Iodid, C₁-C₄-Alkyltosylat oder Mono-C₁-C₄-Alkylsulfat gewählt.

Als Verdünnungsmittel kommen vorzugsweise Ether wie beispielsweise Diethylether, tert. Butylether oder vorzugsweise Dimethoxyethan oder Tetrahydrofuran in Frage. Sofern die Reaktion bei Temperaturen über 12°C durchgeführt wird, kann ferner 1,4-Dioxan eingesetzt werden.

In der Verfahrenvariante a) werden als Lithiumbasen die Lithiumamide der Formel IV eingesetzt, 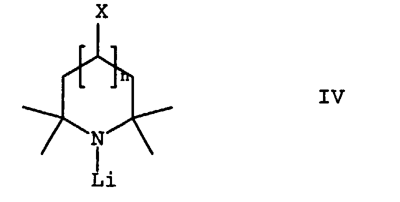 in der n für 0 oder 1 und X für Wasserstoff oder C₁-C₄-Alkoxy stehen. Besonders bevorzugt ist Lithium-2,2,6,6-tetramethylpiperidid und Lithium-4-methoxy-2,2,6,6-tetramethylpiperidid. Die Lithiumamide können durch Umsetzung des jeweiligen Tetramethylpiperidins mit einer lithiumorganischen Verbindung wie beispielsweise Butyllithium hergestellt werden. Vorzugsweise erfolgt die Herstellung der Lithiumbase durch Zutropfen der metallorganischen Verbindung zum im Verdünnungsmittel vorgelegten Amin. In der Regel wird im Anschluß daran das Pyridin I zur vorgelegten Lithiumbase zugetropft. 2,2,6,6-Tetramethylpiperidin ist kommerziell erhältlich, 4-Methoxy-2,2,6,6-tetramethylpiperidin kann durch Methylierung ausgehend von kommerziellem 4-Hydroxy-2,2,6,6-tetramethylpiperidin nach literaturbekannten Verfahren hergestellt werden.

In der Verfahrensvariante b) kann die Lithiumbase Lithiumdiisopropylamid wie oben beschrieben aus Diisopropylamin und beispielsweise Butyllithium hergestellt werden. Man kann jedoch auch von kommerziellem Lithiumdiisopropylamid ausgehen.

Im allgemeinen wird die Lithiumbase in einem Molverhältnis von 1,0 bis 1,7, vorzugsweise von 1,1 bis 1,3 zum Pyridin I eingesetzt.

Das Elektrophil der Formel III bzw. das Alkylierungsmittel der Formel III' wird in der Regel in einem Molverhältnis von 1,0 bis 1,7, vorzugsweise 1,0 bis 1,2 zum Pyridin I eingesetzt.

Die Reaktion wird im allgemeinen bei Temperaturen von -78° bis +20°C und vorzugsweise bei -50° bis 0°C durchgeführt.

Durch selektive Alkylierung sind nach der erfindungsgemäßen Verfahrensvariante a) neue 3-Alkylpyridinen IIa' und nach Variante b) neue 4-Alkylpyridinen IIb' zugänglich.

In Schema 2 ist die selektive Alkylierung beispielhaft ausgehend von 2-Alkoxy-5-trifluormethylpyridin dargestellt. Die Umsetzung mit Lithium-2,2,6,6-tetramethylpiperidid (LTMP) und Methyliodid (MeI) führt zum 2-Alkoxy-3-methyl-5-trifluormethylpyridin (Variante a)) während die Umsetzung mit LDA und MeI nach Variante b) 2-Alkoxy-4-methyl-5-trifluormethylpyridin liefert.

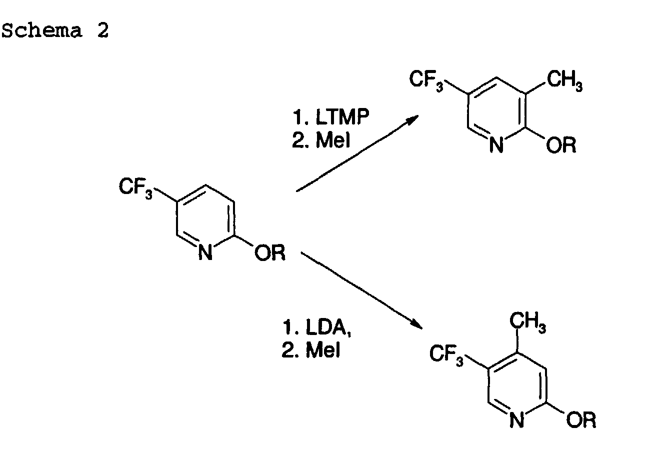

Nach Variante a) sind die erfindungsgemäßen 3-Alkylpyridine der Formel IIa' zugänglich, 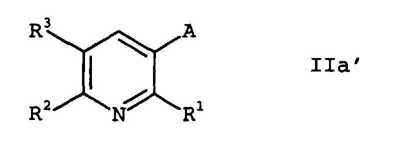 in der die Substituenten die folgende Bedeutung haben:
- R¹: einen Rest OR⁴;
- R²: Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
- R³: perhalogeniertes C₁-C₂-Alkyl oder einen Rest R⁵(C=O)-;
- R⁴: C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
- R⁵: Wasserstoff, perhalogeniertes C₁-C₂-Alkyl, C₁-C₆-Alkoxy oder Di-(C₁-C₄-alkyl)amino und
- A: C₁-C₆-Alkyl.

Nach Variante b) können die erfindungsgemäßen 4-Alkylpyridine der Formel IIb' hergestellt werden, 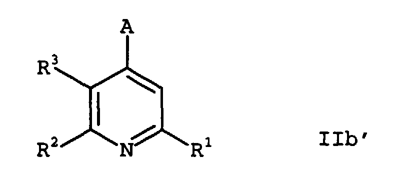 in der die Substituenten die folgende Bedeutung haben:
- R¹: einen Rest OR⁴;
- R²: Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
- R³: perfluoriertes C₁-C₂-Alkyl;
- R⁴: C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
- A: C₁-C₆-Alkyl.

Die für die Substituenten R¹ - R⁵ oder als Reste an Phenylringen genannten organischen Molekülteile der Formeln IIa' und IIb' stellen Sammelbegriffe für individuelle Aufzählungen der einzelnen Gruppenmitglieder dar. Sämtliche Kohlenwasserstoffketten, also alle Alkyl-, Alkoxy-, Alkylcarbonyl-Teile können geradkettig oder verzweigt sein. Die Bedeutung Halogen steht jeweils für Fluor, Chlor, Brom oder Iod, insbesondere für Fluor und Chlor.

Ferner bedeuten beispielsweise:
- C₁-C₄-Alkyl sowie die Alkylteile anderer Reste wie z. B. C₁-C₄-Alkoxy, C₁-C₄-Alkylcarbonyl oder Di-C₁-C₄-alkylamino: Methyl, Ethyl, n-Propyl, 1-Methylethyl, Butyl, 1-Methylpropyl, 2-Methylpropyl und 1,1-Dimethylethyl;
- C₁-C₆-Alkyl sowie die Alkylteile anderer Reste wie z. B. C₁-C₆-Alkoxy, C₁-C₆-Alkylcarbonyl, C₁-C₆-Alkylthio: C₁-C₄-Alkyl wie vorstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- tertiäres C₄-C₆-Alkyl: 1,1-Dimethylethyl, 1,1-Dimethylpropyl, 1,1-Dimethylbutyl, 1,1,2-Trimethylpropyl;
- perhalogeniertes C₁-C₂-Alkyl: einen C₁-C₂-Alkylrest wie vorstehend genannt, der vollständig durch Fluor, Chlor, Brom und/oder Iod substituiert ist, also z. B. Trichlormethyl, Trifluormethyl, Dichlorfluormethyl, Chlordifluormethyl, Pentafluorethyl, insbesondere Trifluormethyl;
- perfluoriertes C₁-C₂-Alkyl: einen C₁-C₂-Alkylrest wie vorstehend genannt, der vollständig durch Fluor substituiert ist, also z. B. Trifluormethyl oder Pentafluorethyl, insbesondere Trifluormethyl.

### Verfahrensbeispiele

### Allgemeine Verfahrensvorschrift (Variante a) und b))

Ein Äquivalent des Amins (für Variante a) beispielhaft 2,2,6,6-Tetramethylpiperidin und für Variante b) Diisopropylamin) wurde in Tetrahydrofuran gelöst. Anschließend wurde bei -70°C ein Äquivalent 1.6 *n* Butyllithium (in *n*-Hexan) zugetropft. Dann wurde bei derselben Temperatur die Pyridinkomponente I zugetropft. Eine Stunde wurde bei -70°C nachgerührt und daraufhin ein Äquivalent des Alkylierungsmittels, gelöst in Tetrahydrofuran, zugegeben, wobei sich die Reaktionsmischung erwärmte. Es wurde eine Stunde bei dieser Temperatur nachgerührt und dann auf -10°C erwärmt. Anschließend wurde mit Wasser hydrolysiert, mit Methyltert.butylether extrahiert und mit Wasser und verd. Salzsäure nachgewaschen. Die vereinigten organischen Phasen wurden über Natriumsulfat getrocknet. Nach Abdestillieren des Lösungsmittels im Vakuum wurde das jeweilige Rohprodukt erhalten, das gegebenfalls durch Säulenchromatographie weiter gereinigt werden konnte.

### Verfahrensbeispiel 1 (Variante a))

### Herstellung von 2-Methoxy-3-methyl-5-trifluormethylpyridin

Ausgehend von 4.7 g (33 mmol) 2,2,6,6-Tetramethylpiperidin, 21 ml (33 mmol) 1.6 *n* Butyllithium in Hexan, 5.3 g (30 mmol) 2-Methoxy-5-trifluormethylpyridin und 4.7 g (33 mmol) Methyliodid wurden 3.2 g (56%) der Titelverbindung erhalten (vgl. EP-A 316 185):
¹H-NMR (CDCl₃): δ (ppm) 2.2 (s, 3 H, CH₃), 3.95 (s, 3 H, OCH₃), 7.5 (s, 1 H, 4-H), 8,25 (s, 1 H, 6-H).

### 3-Alkylpyridine der Formel IIa' (hergestellt nach Variante a))

### Beispiel 2

### 2-Benzyloxy-3-methyl-5-trifluormethylpyridin

Ausgehend von 4.9 g (35 mmol) 2,2,6,6-Tetramethylpiperidin, 22 ml (35 mmol) 1.6 *n* Butyllithium in Hexan, 6.8 g (27 mmol) 2-Benzyloxy-5-trifluormethylpyridin und 5.0 g (35 mmol) Methyliodid wurden 5.5 g (77%) der Titelverbindung erhalten.
¹H-NMR (CDCl₃): δ (ppm) 2.3 (s, 3 H, CH₃), 5.4 (s, 2 H, OCH₂), 7.2-7.4 (bm, 5 H, Ph), 7.5 (s, 1 H, 4-H), 8,25 (s, 1 H, 6-H).

### 4-Alkylpyridine der Formel IIb' (hergestellt nach Variante b))

### 2-Methoxy-4-methyl-5-trifluormethylpyridin

### Beispiel 3

Ausgehend von 1.7 g (17 mmol) Diisopropylamin, 11 ml (17 mmol) 1.6 *n* Butyllithium in Hexan, 2.7 g (15 mmol) 2-Methoxy-5-trifluormethylpyridin und 2.3 g (17 mmol) Methyliodid wurden 1.3 g (45%) der Titelverbindung erhalten.
¹H-NMR (CDCl₃) : δ (ppm) 2.3 (s, 3 H, CH₃), 3.95 (s, 3 H, OCH₃), 6.7 (s, 1 H, 3-H), 8.3 (s, 1 H, 6-H).

## Patentansprüche

1. Verfahren zur selektiven Umsetzung eines Pyridins der Formel I, 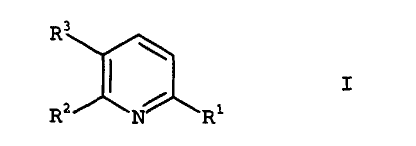 in der die Substituenten die folgende Bedeutung haben:
R¹ einen Rest OR⁴, Chlor oder C₁-C₄-Alkylcarbonylamino;
R² Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
R³ perhalogeniertes C₁-C₂-Alkyl oder einen Rest R⁵(C=O)-;
R⁴ C₁-C₆-Alkyl, C₁-C₄-Alkoxy-C₁-C₂-alkyl, Di-(C₁-C₄-alkyl)aminocarbonyl, Phenyl oder Benzyl, wobei der Phenyl-und Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
R⁵ Wasserstoff, perhalogeniertes C₁-C₂-Alkyl, C₁-C₆-Alkoxy oder Di-(C₁-C₄-alkyl)amino;
mit einen Elektrophil der Formel III,
E-L¹ III
in der E die Bedeutung C₁-C₆-Alkyl, Brom oder Iod hat und L¹ für eine Abgangsgruppe wie Chlorid, Bromid, Iodid oder im Fall, daß E C₁-C₆-Alkyl bedeutet, zusätzlich für C₁-C₆-Alkylsulfonat, Arylsulfonat oder Mono-C₁-C₆-Alkylsulfat steht, zu entweder
a) einen in 3-Stellung substituierten Pyridin der Formel IIa, 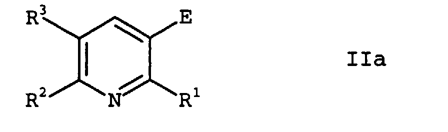 oder
b) einem in 4-Stellung substituierten Pyridin der Formel IIb, 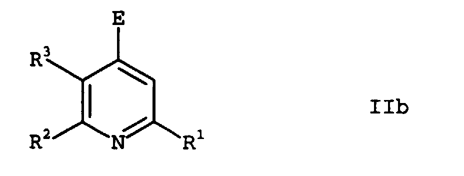 dadurch gekennzeichnet, daß
a) das Pyridin I zunächst mit einer Lithiumbase der Formel IV, 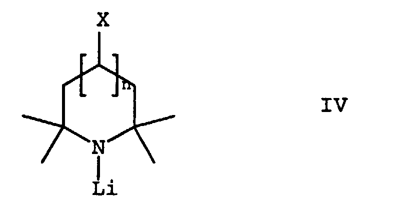 in der n für 0 oder 1 und X für Wasserstoff, C₁-C₄-Alkoxy, Phenoxy oder Benzyloxy stehen, und anschließend mit einem Elektrophil der Formel III zu einem in 3-Stellung substituierten Pyridin der Formel IIa, oder
b) das Pyridin I zunächst mit Lithiumdiisopropylamid und anschließend mit einem Elektrophil der Formel III zu einem in 4-Stellung substituierten Pyridin der Formel IIb
umgesetzt wird.

2. Verfahren nach Anspruch 1 zur selektiven Alkylierung eines Pyridins der Formel I gemäß Anspruch 1, mit einem Alkylierungsmittel der Formel III'
A-L¹ III'
in der A für C₁-C₆-Alkyl und L¹ für eine Abgangsgruppe wie Chlorid, Bromid, Iodid, C₁-C₆-Alkylsulfonat, Arylsulfonat oder Mono-C₁-C₆-Alkylsulfat steht, zu a) einem 3-Alkylpyridin der Formel IIa' 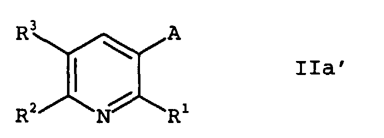 oder b) 4-Alkylpyridin der Formel IIb', 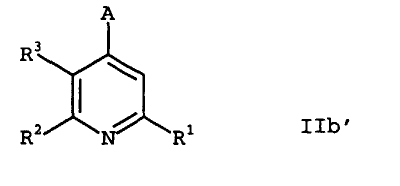 wobei die Substituenten R¹ bis R³ in den Formeln IIa' und IIb' die in Anspruch 1 gegebene Bedeutung haben und der Substituent A jeweils für C₁-C₆-Alkyl steht.

3. Verfahren nach Anspruch 2 zur selektiven Alkylierung eines Pyridins der Formel I 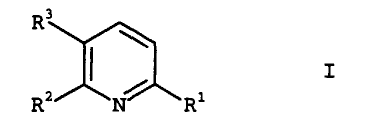 in der die Substituenten die folgende Bedeutung haben:
R¹ einen Rest OR⁴;
R² Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
R³ perfluoriertes C₁-C₂-Alkyl;
R⁴ C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;

4. Verfahren nach Anspruch 2, Variante a) zur selektiven Alkylierung eines Pyridins der Formel I, in der die Substituenten die folgende Bedeutung haben:
R¹ einen Rest OR⁴;
R² Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
R³ perhalogeniertes C₁-C₂-Alkyl oder einen Rest R⁵(C=O)-;
R⁴ C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
R⁵ Wasserstoff, perhalogeniertes C₁-C₂-Alkyl, C₁-C₆-Alkoxy oder Di-(C₁-C₄-alkyl)amino;
zu 3-Alkylpyridinen IIa'.

5. Verfahren nach einem der Ansprüche 1 bis 4, dadurch gekennzeichnet, daß das Verfahren in einem etherischen Verdünnungsmittel durchgeführt wird.

6. 3-Alkylpyridine der Formel IIa', 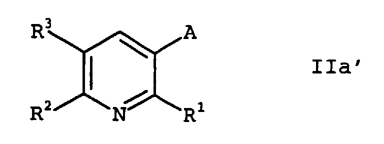 in der die Substituenten die folgende Bedeutung haben:
R¹ einen Rest OR⁴;
R² Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
R³ perhalogeniertes C₁-C₂-Alkyl oder einen Rest R⁵ (C=O)-;
R⁴ C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
R⁵ Wasserstoff, perhalogeniertes C₁-C₂-Alkyl, C₁-C₆-Alkoxy oder Di-(C₁-C₄-alkyl)amino und
A C₁-C₆-Alkyl.

7. 4-Alkylpyridine der Formel IIb', 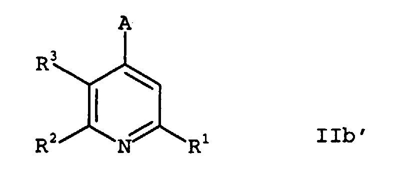 in der die Substituenten die folgende Bedeutung haben:
R¹ einen Rest OR⁴;
R² Wasserstoff, Fluor, Chlor, tertiäres C₄-C₆-Alkyl, perhalogeniertes C₁-C₂-Alkyl, einen Rest OR⁴ oder Phenyl, welches unsubstituiert oder ein bis drei der folgenden Substituenten: C₁-C₆-Alkyl, C₁-C₆-Alkoxy, Di-(C₁-C₄-alkyl)amino, Aryl, Halogen oder Nitro tragen kann;
R³ perfluoriertes C₁-C₂-Alkyl;
R⁴ C₁-C₆-Alkyl, C₁-C₄-Alkoxy-C₁-C₂-alkyl, Di-(C₁-C₄-alkyl)aminocarbonyl, Benzyl, wobei der Benzylrest im Phenylteil ein bis drei Substituenten ausgewählt aus der Gruppe: C₁-C₆-Alkyl, C₁-C₆-Alkoxy, Di-(C₁-C₄-alkyl)amino, Aryl, Halogen oder Nitro tragen kann;
A C₁-C₆-Alkyl.
